# EUROPÄISCHE PATENTSCHRIFT

(11) **EP 0 746 334 B1**
(45) Veröffentlichungstag und Bekanntmachung des Hinweises auf die Patenterteilung: **05.09.2001**
(21) Anmeldenummer: 95910500.8
(22) Anmeldetag: 18.02.1995
(51) Int. Cl.: A61K 38/49, A61K 47/18, A61K 47/26

(54) **PHARMAZEUTISCHES PRÄPARAT ENTHALTEND PLASMINOGENAKTIVATOREN**
PHARMACEUTICAL PREPARATION CONTAINING PLASMINOGEN ACTIVATORS
PREPARATION PHARMACEUTIQUE CONTENANT DES ACTIVATEURS DU PLASMINOGENE

(30) Priorität: 21.02.1994 DE 4405426
(43) Veröffentlichungstag der Anmeldung: 11.12.1996
(73) Patentinhaber: Roche Diagnostics GmbH, 68298 Mannheim (DE)
(72) Erfinder: KOHNERT, Ulrich, D-82392 Habach (DE); FISCHER, Stephan, D-82938 Polling (DE); MARKL, Hans-Jörg, D-67158 Ellerstadt (DE); WOOG, Heinrich, D-69514 Laudenbach (DE)
(74) Vertreter: Schreiner, Siegfried, Dr.
(86) Internationale Anmeldenummer: EP9500596
(87) Internationale Veröffentlichungsnummer: WO9522347

(56) Entgegenhaltungen:
- EP-A- 0 297 294
- WO-A-91/08766
- DE-A- 3 942 141
- DE-A- 3 942 144

## Beschreibung

Die Erfindung betrifft pharmazeutische Präparate, die als Wirkstoffe Plasminogen-aktivatoren oder deren Derivate enthalten, sowie entsprechende pharmazeutische Darreichungsformen in Form von Lyophilisaten oder Injektions- bzw. Infusionslösungen.

Der menschliche Gewebe-Plasminogenaktivator (t-PA) besitzt eine große therapeutische Bedeutung bei der Auflösung von Blutgerinnseln, z. B. bei Herzinfarkten. t-PA bewirkt die Auflösung von Blutgerinnseln durch die Aktivierung von Plasminogen zu Plasmin. Plasmin wiederum löst Fibrin, die Hauptkomponente der Proteinmatrix von geronnenem Blut.

Natürlicher t-PA ist aus mehreren funktionellen Domänen F, E, K1, K2 und P zusammengesetzt. Die Domäne P beinhaltet das proteolytisch aktive Zentrum, das die Spaltung von Plasminogen zu Plasmin bewirkt. Die gentechnologische Herstellung von Plasminogen-aktivatoren (PA), insbesondere von t-PA oder verschiedenen t-PA-Muteinen in eukaryontischen und prokaryontischen Zellen ist bereits bekannt. Dabei werden t-PA-Derivate aus Prokaryonten im Gegensatz zu natürlichem t-PA in nicht-glykosylierter Form synthetisiert.

Als Plasminogenaktivatoren kommen im Sinne der Erfindung prinzipiell solche, insbesondere rekombinant hergestellte Derivate des t-PA in Frage, die im wesentlichen diejenigen Proteinbereiche des natürlichen Proteins umfassen, die für die Fibrinolyse der Thromben zuständig sind. Dabei können auch solche Derivate des t-PA verwendet werden, die Deletionen oder Substitutionen einzelner oder mehrerer Aminosäuren in der Sequenz des t-PA aufweisen.

Erfindungsgemäß können beispielhaft die folgenden Plasminogenaktivatoren eingesetzt werden: t-PA (z.B. Alteplase), LY 210825 (K2P erhältlich aus Zelllinien des "syrian hamster", Circ. 1990, 82, 930-940); ΔFE3x und ΔFE1x (K1K2P, Blood 1988, 71, 216-219); ΔFEK1 (K2P aus C127 Mäusezellen, J. Cardiovasc. Pharmacol. 1990, 16, 197209); E-6010 (Jap. Circ. J. 1989, 53, p. 918); t-PA-Varianten (Thromb. Haemost., 1989, 62, p. 542); K2P und D-K2P (Thromb. Haemost., 1989, 62, p. 393); MB-1018 (FK2K2P, Thromb. Haemost., 1989, 62, p. 543); FK2P (FASEB J., 1989, 3, A1031, abstract 4791); Δlx (Circulation, 1988, 4, II-15); K1K2P (Thromb. Res., 1988, 50, 33-41); FK1K2P (J. Biol. Chem., 1988, 263, 1599-1602); TNK-Variante von t-PA (WO 93/24635); bat-PA (Witt et al., Blood 1992; 79: 1213-1217 und Mullot et al., Arterioscler. Thrombos. 1992; 12: 212-221). Insbesondere kommen solche Plasminogenaktivatoren in Frage, die die Kringel-2-Domäne ("K2") des t-PA und/oder die Serin-Protease-Domäne ("P") enthalten. Beispielhaft sei diesbezüglich das t-PA-Mutein "r-PA" vom Typ K2P genannt, das in der EP 0 382 174 (WO 90/09437) beschrieben ist.

Die vorliegende Erfindung betrifft insbesondere Plasminogenaktivatoren vom Typ K2P, K1K2P, FK1K2P und FK2K2P, wie sie in folgenden Schriften beschrieben sind: Protein Engineering 5(1), 93-100 (1992); DE-OS-39 23 339.1; Circ. 1990, 82, 930-940; J. Cardiovasc. Pharmacol. 1990, 16 ,197-203; Blood 1988, 71, 216-219; J. Biol. Chem. 1988, 263, 1599-1602; Thromb. Haemost. 1989, 62, S. 543. Insbesondere werden rekombinante Plasminogenaktivatoren vom Typ K2P eingesetzt, wie sie in EP-A-0 382 174 und in Protein Engineering Vol. 5(1), S. 93-100 (1992) beschrieben sind. Weitere t-PA-Muteine dieser Art sind in den folgenden Patentanmeldungen beschrieben: US 4,970,159, EP-A-0,196,920, EP-A-0,207,589; AU 61804/86; EP-A-0,231,624; EP-A-0,289,508; JP 63133988; EP-A-0,234,051; EP-A-0,263,172; EP-A-0,241,208; EP-A-0,292,009; EP-A-0,297,066; EP-A-0,302,456; EP-A-0,379,890.

Aus dem Stand der Technik ist bekannt, daß der Zuckeranteil einen erheblichen Einfluß auf die Löslichkeit und Aggregation von Proteinen hat (J. Biol. Chem. 263 (1988), 8832-8837). So wurde in EP-B-458 950 festgestellt, daß z. B. nicht-glykosylierter rekombinanter Plasminogenaktivator mit der Domänen-Zusammensetzung K2P eine wesentlich schlechtere Löslichkeit besitzt als etwa glykosylierte t-PA-Derivate. Nicht-glykosylierte Plasminogenaktivatoren, wie z.B. r-PA, lösen sich in der Regel nur in geringem Ausmaß in den üblicherweise zur Solubilisierung von Proteinen verwendeten Puffern, wie z. B. 50 mmol/l Na-Citrat pH 6, 50 mmol/l Phosphat-Puffer oder physiologischer NaCl-Lösung. Für den Einsatz als therapeutischer Wirkstoff ist es jedoch erforderlich, daß Plasminogenaktivatoren in ausreichend hohen Konzentrationen, vorzugsweise in einer Konzentration bis zu 10 mg/ml vorliegen.

Aus der EP-A-0 217 379 ist bekannt, die Löslichkeit von t-PA aus Prokaryonten durch neutrale oder leicht alkalische Arginin-Formulierungen zu erhöhen. Ein Nachteil dieses Verfahrens ist jedoch, daß gute Löslichkeiten von t-PA aus Prokaryonten nur mit sehr hohen Arginin-konzentrationen erreicht werden können.

Weitere galenische Formulierungen von Plasminogenaktivatoren oder deren Derivate sind aus WO 90/01333, WO 89/050347, WO 90/08557, EP 0 297 294, EP 0 156 169 und EP 0 228 862 bekannt.

So wird in WO 90/01333 (Invitron) die Kombination von Lysin, Histidin, Arginin mit Citrat für t-PA und Derivate aus Bakterien beschrieben. Citrat wird mit 5 mmol/l, Lysin, Histidin, Arginin mit 150 mmol/l bei pH 6 eingesetzt. Außerdem wird Albumin zugesetzt.

In WO 89/050347 (Invitron) wird die Kombination von Arginin (20 - 200 mmol/l) und Citrat (20 - 80 mmol/l) bei pH 5 - 8 beschrieben.

WO 90/08557 (Genetics Institute) offenbart eine Kombination von Creatinin mit verschiedenen Zusatzstoffen wie Histidin, Arginin, Prolin, Betain, Collin, Imidazol, Tryptophan, Citrat, gegebenenfalls unter Zusatz von Glutaminsäure, Asparaginsäure und Bernsteinsäure.

In EP 0 297 294 (Behring) wird die Kombination von mindestens zwei Aminosäuren wie Lysin, Ornithin, Arginin, Tranexamsäure und anderen Zusatzstoffen bei pH 5 - 10 offenbart.

EP 0 156 169 (Asahi) beschreibt Ornithin und/oder Lysin, gegebenenfalls unter Zusatz von Citrat, Glycin oder Phosphat und EP 0 228 862 (Genentech) offenbart eine Formulierung enthaltend Arginin mit oder ohne Chlorid sowie mit oder ohne Detergenz.

Alternative Formulierungen von r-PA - in Gegenwart von Lysin bzw. Lysin-Analoga in Citronensäure-gepufferten Lösungen werden auch in EP 0 458 950 (Boehringer Mannheim) beschrieben. Neuere Untersuchungen zeigen jedoch, daß die Löslichkeit von r-PA auch in diesen Formulierungen noch nicht völlig ausreichend ist. Es wurde festgestellt, daß sich die Löslichkeit zwar durch Erhöhung der Citrat-Konzentration verbessern läßt, jedoch sind derartige Formulierungen nicht oder nur bedingt venenverträglich. Daneben führen die hohen Salzkonzentrationen und die damit verbundene niedrige Glastemperatur (Tg') dazu, daß die Lyophilisation dieser Formulierungen bei Temperaturen unterhalb von -45 °C und -50 °C durchgeführt werden muß. Diese Temperaturen sind technisch oft nur mit einem sehr hohen Aufwand realisierbar und erfordern aufwendige Steuer- und Überwachungselemente, um die optimalen Bedingungen für die Lyophilisation zu messen und zu regeln. Außerdem ist damit ein relativ hoher Energieaufwand verbunden. Hinzu kommt, daß häufig für die großtechnische Produktion ältere Lyophilisationsanlagen verwendet werden, die nicht über die erforderliche komplizierte Meß- und Regeltechnik verfügen, und bei denen demzufolge in der Regel nur eine Arbeitstemperatur von ca. -45°C garantiert werden kann.

Aufgabe der Erfindung ist es, Formulierungen von Plasminogenaktivatoren und deren Derivaten bereitzustellen, die gut venenverträglich sind, den Wirkstoff in ausreichend hohen Konzentrationen enthalten und eine gute Löslichkeit des Wirkstoffes gewährleisten, wobei die Stabilität des PA im Lyophilisat über einen längeren Zeitraum hinweg erhalten bleiben soll. Außerdem ist es das Ziel der Erfindung, Formulierungen zu entwickeln, die auch im technischen Produktionsmaßstab sicher lyophilisierbar sind, womit eine gut reproduzierbare Produktqualität der Lyophilisate gewährleistet sein soll.

Die erfindungsgemäße Aufgabe wird durch ein einen Plasminogenaktivator enthaltendes pharmazeutisches Präparat gelöst, wobei das Präparat als pharmazeutische Zusatzstoffe mindestens einen Zucker und Tranexamsäure enthält. Das Präparat ist in lyophilisierter Form über einen längeren Zeitraum hinweg lagerstabil. Auch die durch Rekonstition hergestellte wässrige Injektionslösung erfüllt dieses Kriterium der erhöhten Lagerstabilität. Dies ist insbesondere dann der Fall, wenn der pH-Wert der Lösung auf einen Wert zwischen 5,5 - 6,5 eingestellt wird. Als weitere Zusatzstoffe kann das pharmazeutische Präparat übliche Puffersubstanzen oder oberflächenaktive Stoffe (anionische, kationische oder neutrale Tenside) enthalten.

Beispielhaft für die im Sinne der vorliegenden Erfindung in Frage kommenden Plasminogenaktivatoren (PA) wurde der in der Europäischen Patentanmeldung EP-A-0,382,174 näher beschriebene Plasminogenaktivator K2P (BM 06.022) verwendet. Er besteht aus dem Kringel 2 (K2) und der Protease-Domäne (P) des humanen t-PA und liegt wegen seiner Expression in Escherichia-coli-Zellen in unglykosylierter Form vor.

Als Zucker enthalten die erfindungsgemäßen Formulierungen vorzugsweise Mono- oder Disaccharide. Als Disaccharide kommen insbesondere Saccharose, Trehalose, Maltose oder Lactose in Frage. Monosaccharide sind insbesondere Galactose oder die entsprechenden Aminozucker, wie z.B. Galactosamin. Bevorzugt werden die nicht-reduzierenden Zucker Saccharose oder Trehalose eingesetzt. Die Konzentration des Zuckers in der wässrigen Injektionslösung beträgt vorzugsweise 40 - 100 mg/ml, bevorzugt liegt sie zwischen 50 - 70 mg/ml.

Als Puffersubstanzen können die pharmazeutischen Präparate übliche für diese Zwecke in Frage kommende Substanzen enthalten, die Salze von starken Säuren mit schwachen Basen oder von schwachen Säuren mit starken Basen darstellen. Beispielhaft seien erwähnt: Alkalisalze von Phosphorsäure, Weinsäure, Äpfelsäure und dergleichen. Ebenso können Aminosäuren in Frage kommen. Bevorzugt enthalten die erfindungsgemäßen Präparate Phosphatpuffer. Die Konzentration von Phosphatpuffer in der fertig injizierbaren wässrigen Lösung beträgt insbesondere 50 - 300 mMol/l, bevorzugt 80 - 220 mMol/l, und insbesondere bevorzugt von 130 - 170 mMol/l. Als Phosphatpuffer wird vorzugsweise Dinatrium- oder Dikaliumhydrogenphosphat verwendet, das mit Phosphorsäure auf den jeweiligen pH-Wert eingestellt wird.

Als Löslichkeitsvermittler enthalten die erfindungsgemäßen Präparate Tranexamsäure (TES). Die Konzentration von Tranexamsäure in der fertig injizierbaren wässrigen Lösung beträgt bevorzugt 1 - 50 mMol/l, vorzugsweise von 8 - 12 mMol/l. Besonders vorteilhaft wird eine Konzentration von 10 mMol/l verwendet. Überraschenderweise wurde gefunden, daß Tranexamsäure die Löslichkeit von Plasminogenaktivatoren, insbesondere von nicht-glykosylierten Plasminogenaktivatoren, in wässrigem Medium erhöht. Beispielsweise wurde im Falle des Plasminogenaktivator-Derivates r-PA die Löslichkeit mindestens um den Faktor 1,5 gegenüber einer tranexamsäure-freien Lösung erhöht. Der Faktor für die Löslichkeitserhöhung liegt vorzugsweise im Bereich von 2 - 3. Dieser Effekt ist vor allem im Rahmen der Herstellung von lyophilisierten pharmazeutischen Zubereitungen von Plasminogenaktivatoren von Vorteil, da durch die Möglichkeit der Konzentrationserhöhung des Plasminogenaktivators in den verwendeten Lösungen die im Herstellungsprozeß zu verwendende Wassermenge deutlich reduziert werden kann und insgesamt eine energiesparende und kostengünstigere Herstellung erzielt wird. Insbesondere verkürzen sich hierdurch die Lyophilisationszeiten beim Herstellungsprozeß.

Als Tenside können nicht-ionische, anionische oder kationische Tenside, vorzugsweise jedoch nicht-ionische Tenside, wie beispielsweise Tween 80® oder Tween 20®, eingesetzt werden. Die Tensidkonzentration beträgt 0,005 - 0,1 % (w/v), vorzugsweise 0,01 %.

Geeignet für ein erfindungsgemäßes pharmazeutisches Präparat in wässriger Form ist ein pH-Wert zwischen 5,5 - 6,5, bevorzugt ist ein pH-Wert von 5,8 - 6,2.

Die erfindungsgemäßen Präparate enthalten den Wirkstoff in einer Konzentration bis zu 10 mg/ml, vorzugsweise von 3 - 5 mg/ml.

Die erfindungsgemäßen pharmazeutischen Präparate kommen als Injektions- oder Infusionslösungen zum Einsatz. Dies kann dadurch geschehen, daß eine bereits spritzfertige Lösung zur Verfügung gestellt wird, die die erfindungsgemäße Zusammensetzung besitzt. Es ist jedoch auch möglich, die pharmazeutischen Präparate in Form von Lyophilisaten zur Verfügung zu stellen. Diese werden dann mit an sich bekannten, zu Injektionszwecken geeigneten Mitteln oder Lösungen (z. B. mit Wasser) rekonstituiert. Als Injektionsmedium kommt für die erfindungsgemäßen Präparate vorzugsweise Wasser zur Anwendung, welches gegebenenfalls übliche isotonische Zusätze wie beispielsweise eine physiologische NaCl-Konzentration enthalten kann.

Gegenstand der Erfindung sind auch das Verfahren zur Herstellung eines pharmazeutischen Präparats enthaltend Plasminogenaktivatoren oder deren Derivate mit einem pH-Wert von 5,5 bis 6,5 sowie deren Verwendung zur Herstellung der erfindungsgemäßen pharmazeutischen Präparate.

Gegenstand der Erfindung ist außerdem die Verwendung einer bestimmten Mischung von pharmazeutischen Hilfsstoffen bestehend aus der Gruppe Zucker und Tranexamsäure zur Langzeitstablilisierung von Plasminogenaktivatoren. Besonders vorteilhaft ist insbesondere eine Kombination der Hilfsstoffe Zucker, Phophatpuffer, Tranexamsäure und Tensid zur Erzielung einer guten Lagerstabilität.

Stabilitätsuntersuchungen zeigen, daß die erfindungsgemäßen Präparate als Lösungen bei 4 °C mindestens 30 Tage stabil sind. Die Stabilität des Wirkstoffes in den erfindungsgemäßen Lyophilisaten beträgt bei 4 °C zwischen zwei und fünf Jahren. Vorteilhaft wirkt sich insbesondere die Tatsache aus, daß die Arnzeimittelpräparate auch bei höherer thermischer Belastung eine ausgezeichnete Stabilität aufweisen, die sie weitgehend unempfindlich macht gegenüber Temperaturschwankungen, die insbesondere in wärmeren Klimaregionen der Erde, insbesondere bei einer Unterbrechung der Kühlkette auftreten können. Aufgrund der oft langen Vertriebswege im Falle von pharmazeutischen Darreichungsformen vom Arzneimittelhersteller bis zum Endverbraucher in den verschiedenen Ländern kann nicht ausgeschlossen werden, daß die Präparate unbeabsichtigt gegebenenfalls auch über einen längeren Zeitraum hinweg Temperaturschwankungen ausgesetzt sind, die zu einem Aktivitätsverlust des Proteins führen, und somit den therapeutischen Behandlungserfolg im Extremfall in Frage stellen. Die erfindungsgemäßen Darreichungsformen sind jedoch gegenüber solchen Temperaturschwankungen weitgehend unempflindlich. In vorläufigen Stabilitätsuntersuchungen konnte gezeigt werden, daß bei den Präparaten selbst bei einer Temperatur von 35 °C, die sie für einen Zeitraum von 30 Tagen ausgesetzt waren, keine nennenswerte Beeinträchtigung hinsichtlich der Stabilität des Proteins festgestellt werden konnte. Es ist demzufolge davon auszugehen, daß die Präparate bei ordnungsgemäßer Lagerung bei Kühlschranktemperatur eine Stabilität von zwei bis fünf Jahren aufweisen werden.

Die erfindungsgemäßen Präparate haben außerdem den Vorteil, daß die Glastemperatur (Tg') der eingefrorenen Lösung zur Herstellung der Lyophilisate relativ hoch ist und zwischen -33 °C und - 40 °C liegt, so daß die Reproduzierbarkeit der Produktqualität der Lyophilisate auch im Produktionsmaßstab mit hinreichender Sicherheit gewährleistet ist. Die relativ hohe Glastemperatur ist insbesondere deshalb von Vorteil, da bei technisch bedingten oder unvorhergesehenen Temperaturerhöhungen während der oft sehr langen Lyophilisationszeiten das Risiko eines unbeabsichtigten Auftauens der eingefrorenen Lösungen weniger wahrscheinlich ist. In Fällen, in denen die Glastemperatur unterhalb von -40 °C liegt und diese Temperatur während des Lyophilisationsprozeßes nach oben hin überschritten wird, was insbesondere bei technischen Lyophilisationsanlagen erfolgt, die bei Temperaturen in der Nähe von -45 °C arbeiten, kann das unerwünschte Überschreiten der Glastemperatur zu nachteiligen Veränderungen der gefrorenen Lösung führen. Eine ausreichend gute Produktqualität ist dann nicht mehr gewährleistet. Es kann dabei insbesondere bei proteinhaltigen Lyophilisaten zum Aktivitätsverlust des Proteins kommen, bzw. zur Bildung von Aggregaten. Ein weiterer Vorteil bei den erfindungsgemäßen Präparaten mit einer Glastemperatur von -33 °C bis - 40 °C besteht darin, daß der erforderliche Energieaufwand für den Lyophilisationsprozeß minimiert wird, da der Lyophilisationsvorgang nicht notwendigerweise bei Temperaturen unterhalb von -50 °C durchgeführt werden muß.

Im Sinne der vorliegenden Erfindung lassen sich insbesondere dann relativ hohe Glastemperaturen für die eingefrorenen Lösungen erzielen, wenn als Phosphatpuffer für die Lyophilisation erforderlichen Lösungen ein Kaliumsalz, wie zum Beispiel Dikaliumhydrogenphosphat oder Kaliumdihydrogenphosphat verwendet wird. Insbesondere kommt diesbezüglich Dikaliumhydrogenphosphat in einer Konzentration von 20- 40 mg/ml, vorzugsweise von 20 - 30 mg/ml in Frage. Die Erhöhung der Glastemperatur läßt sich durch den Zusatz von Saccharose verstärken. Saccharose wird hierbei vorzugsweise in einer Konzentration von 60 - 90 mg/ml, insbesondere 60 - 80 mg/ml zugesetzt. Besonders bevorzugt wird eine Lösung verwendet, die etwa 26 mg/ml Dikaliumhydrogenphosphat (K₂HPO₄ x 12 H₂O) und etwa 70 mg/ml Saccharose enthält. Der pH-Wert der Lösung wird vorzugsweise mit 85-%iger Phosphorsäure (etwa 12 mg/ml) auf einen Wert von 6 eingestellt. Weiterhin enthält die Lösung etwa 0,5 - 5 mg/ml, bevorzugt 1 - 2 mg/ml Tranexamsäure, insbesondere etwa 1,6 mg/ml. Außerdem können ferner Tenside enthalten sein, wie zum Beispiel Tween 80®, das bevorzugt in einer Konzentration von 0,01 - 0,3 mg/ml, insbesondere etwa 0,1 mg/ml eingesetzt wird.

Ein weiterer Vorteil der erfindungsgemäßen Lyophilisate besteht darin, daß sie eine relativ geringe Restfeuchte nach Abschluß der Lyophilisation aufweisen. Die Restfeuchte liegt insbesondere bei Werten von weniger als 5 %, vorzugsweise zwischen 0,5 - 4 %, insbesondere 1 - 3 %. Üblicherweise liegen die Werte für Lyophilisate bei Werten oberhalb von 6 %. Die niedrige Restfeuchte trägt dazu bei, daß die Präparate über eine bessere Lagerstabilität verfügen. Präparate mit höherer Restfeuchte führen häufig zu einer Instabilität des Proteins, die sich durch Verlust an biologischer Aktivität oder durch Bildung von Aggregaten bemerkbar macht.

Ein weiterer Vorteil der erfindungsgemäßen Präparate besteht darin, daß bei der Herstellung der Lyophilisate aufgrund der durch den Zusatz von Tranexamsäure erzielten Löslichkeitserhöhung von nicht-glykosylierten Plasminogenaktivatoren, insbesondere im Fall der Muteine vom Typ K2P, K1K2P oder P, von kleineren Volumina (beispielsweise von ca 5 ml pro Einzeldarreichungsform) der einzufrierenden Lösungen ausgegangen werden kann, so daß sich die Lyophilisationszeit gegenüber den bisher zur Herstellung von Lyophilisaten verwendeten Lösungen (beispielsweise von ca. 10 ml pro Darreichungsform) deutlich verkürzt. Vorzugsweise geht man von Lösungen aus, die den Wirkstoff in einer ungefähr zweifach höheren Konzentration im Vergleich zu der injektionsfertigen wässrigen Darreichungsfomen enthalten, so daß anstelle der bisher üblichen Lösungen von 10 ml wässrige Lösungen von 5 ml eingesetzt werden können.

Untersuchungen zur Venenverträglichkeit zeigen eine sehr gute Verträglichkeit der erfindungsgemäßen Präparate.

Die nachfolgenden Ausführungsbeispiele sollen die Erfindung näher erläutern, ohne sie einzuschränken:

### Beispiel 1:

### Eintrübung nach mechanischer Belastung / Messung der Lichtstreuung erfindungsgemäßer Präparate

r-PA (BM 06.022) wurde auf eine Proteinkonzentration (C_{Prot.}) von 6 mg/ml eingestellt (Ultrafiltration über eine Amicon YM 10-Membran) und gegen die angegebenen Puffer dialysiert. Anschließend wurden die Proben auf C_{Prot.} = 4 mg/ml eingestellt und a) unverändert gelassen, b) mit 0.01 % Tween 80® und c) mit 0.01 % Tween 20® aufgestockt.

Die Belastung der Proben erfolgte jeweils für 10 s auf einem Whirl Mix (Janke & Kunkel, IKA-Labortechnik VF2, maximale Drehzahl). Anschließend wurden die Proben für 2 Minuten bei Raumtemperatur inkubiert.

Die Lichtstreuung der unbelasteten und der belasteten Probe wurde bei 25 °C fluorimetrisch gemessen (Ex. 360 nm, Em.: 360 nm; Ex.-bandpath: 3 nm; Em.-bandpath: 10 nm; Meßintervall: 10 s über 3 min). Bestimmt wurde jeweils der um die Fluoreszenz des Puffers korrigierte Wert für die Streuung der Probe.

Ergebnis: Es konnte gezeigt werden, daß es ohne Zusatz von Detergentien unter mechanischer Belastung zu einem erheblichen Anstieg der Lichtstreuung der Probe kommt. Der Anstieg kann durch den Zusatz von Detergentien weitesgehend unterdrückt werden. Im Rahmen der Meßgenauigkeit ist kein Unterschied zwischen Tween 80® und Tween 20® zu erkennen.

Verwendete Pufferlösungen
A) 150 mM Na₂HPO₄/H₃PO₄, pH 6.0
   10 mM TES
   50 mg/ml Saccharose
   a) ohne Detergenz
   b) mit 0.01 % Tween 80®
   c) mit 0.01 % Tween 20®
B) 150 mM Na₂HPO₄/H₃PO₄, pH 6.0
   10 mM TES
   50 mg/ml Trehalose
   a) ohne Detergenz,
   b) mit 0.01 % Tween 80®,
   c) mit 0.01 % Tween 20®
C) 150 mM K₂HPO₄/H₃PO₄, pH 6.0
   10 mM TES
   50 mg/ml Saccharose
   a) ohne Detergenz,
   b) mit 0.01 % Tween 80®,
   c) mit 0.01 % Tween 20®
D) 150 mM K₂HPO₄/H₃PO₄, pH 6.0
   10 mM TES
   50 mg/ml Trehalose
   a) ohne Detergenz,
   b) mit 0.01 % Tween 80®,
   c) mit 0.01 % Tween 20®

### Beispiel 2:

### Lagerung von r-PA (BM 06.022) in TES / Saccharose-haltigen erfindungsgemäßen Formulierungen // Mehrmaliges Einfrieren und Auftauen

r-PA wurde gegen den in Tabelle 1 angegebenen Puffer (ohne Tween 80) dialysiert, auf C_{Prot.} = 4 mg/ml eingestellt, mit Tween 80® auf C = 0.01 % aufgestockt, portioniert und sterilfiltriert. Jeweils 9 Proben wurden bei - 20 bzw. - 70 °C eingefroren. An den in der Tabelle angegebenen Tagen wurden bis auf die Kontrolle alle Proben aufgetaut (15 min bei 25 °C im Wasserbad). Jeweils eine Probe wurde analysiert (C_{Prot.} und amidolytische Aktivität). Die restlichen Proben wurden wieder bei - 20 bzw. - 70 °C tiefgefroren. Nach Abschluß der Serie wurde die Aktivität der nicht gestreßten Kontrolle bestimmt.

Ergebnis: Wie anhand der Daten aus Tabelle 1 hervorgeht, kann r-PA ohne Verlust an Aktivität mindestens acht mal eingefroren und aufgetaut werden.

**Tabelle 1**

| 150 mM Na₂HPO₄/H₃PO₄, pH 6.0 10 mM TES 50 mg/ml Saccharose 0.01 % Tween 80® | | | | |
|---|---|---|---|---|
| | -20 °C | | -70 °C | |
| Tag | C_{Prot.} [mg/ml] | AA [MU/ml] | CProt. [mg/ml] | AA [MU/ml] |
| 0 | 4,1 | 3,1 | 4,1 | 3,1 |
| 1 | 4,5 | 3,1 | 4,6 | 3,0 |
| 2 | 4,6 | 3,2 | 4,8 | 3,1 |
| 3 | 4,3 | 3,1 | 4,0 | 3,0 |
| 4 | 4,8 | 3,1 | 4,7 | 3,1 |
| 5 | 4,1 | 3,3 | 4,1 | 3,2 |
| 6 | 4,2 | 3,0 | 4,2 | 3,1 |
| 7 | 4,1 | 3,1 | 4,3 | 3,2 |
| 8 | 4,0 | 2,9 | 4,3 | 3,1 |
| Kontrolle | 4,3 | 3,0 | 4,3 | 3,1 |
| Kontrolle: Probe unbehandelt vermessen. | | | | |
| AA: Amidolytische Aktivität C_{Prot.}: Proteinkonzentration | | | | |

### Beispiel 3:

### Stabilität von r-PA in Lösung Vergleich verschiedener Formulierungen bei C_{Prot.} = 4 mg/ml

r-PA (BM 06.022) wurde über eine Amicon YM 10-Membran auf 5 mg/ml konzentriert und gegen den in Tabelle 2 angegebenen Puffer (ohne Tween 80®) dialysiert. Die Dialysate wurden auf C_{Prot.} = 4 mg/ml eingestellt, mit Tween 80® auf C = 0.01 % aufgestockt, portioniert und bei - 20 und 4 °C gelagert. Nach 7, 14, 20 und 30 Tagen wurden die amidolytische Aktivität und die Proteinkonzentration der belasteten Proben bestimmt.

Ergebnis: Die bei - 20 und 4 °C gelagerten Proben bleiben über 30 Tage unverändert.

**Tabelle 2**

| 150 mM Na₂HPO₄/H₃PO₄, pH 6.0 10 mM TES 50 mg/ml Trehalose 0.01 % Tween 80® | | | | |
|---|---|---|---|---|
| | -20 °C | | +4°C | |
| Tag | C_{Prot.} [mg/ml] | AA [MU/ml] | C_{Prot.} [mg/ml] | AA [MU/ml] |
| 0 | 3,9 | 2,1 | | |
| 7 | 3,9 | 2.3 | 3,97 | 2,4 |
| 14 | 3,9 | 2,3 | 3,98 | 2,4 |
| 20 | 3,9 | 2,3 | 3,9 | 2,4 |
| 30 | 3,9 | 2,3 | 3,9 | 2,4 |
| AA: Amidolytische Aktivität C_{Prot.}: Proteinkonzentration | | | | |

### Beispiel 4:

### Stabilität von r-PA in Lösung Vergleich verschiedener Formulierungen bei C_{Prot.} = 6 mg/ml

r-PA (BM 06.022) wurde über Nacht gegen die unten aufgeführten Puffer dialysiert und durch Konzentrieren über eine Amicon YM 10-Membran auf C_{Prot.} = 6 mg/ml eingestellt. Die Proben wurden in 1 ml-Portionen abgefüllt und über 30 Tage bei - 20 und 4 ° C gelagert. Nach 1, 2, 5, 9, 15 und 29 Tagen wurden jeweils die Aktivität und der Proteingehalt bestimmt.

**Tabelle 3**

| 200 mM Na₂HPO₄/H₃PO₄, pH 6.0 10 mM TES 50 mg/ml Saccharose 0.01 % Tween 80 | | | | |
|---|---|---|---|---|
| | -20 °C | | + 4 °C | |
| Tag | CProt. [mg/ml] | AA [MU/ml] | C_{Prot.} [mg/ml] | AA [MU/ml] |
| 0 | 6,0 | 3,3 | 6,0 | 3,3 |
| 1 | 5,7 | 3,3 | 5,7 | 3,6 |
| 2 | 5,8 | 3.3 | 5,9 | 3,4 |
| 5 | 5,9 | 3.2 | 6,0 | 3,6 |
| 9 | 6,0 | 3,3 | 5,9 | 3,7 |
| 15 | 5,8 | 3,5 | 5,9 | 3,5 |
| 29 | 5,9 | 3,6 | 5,9 | 3,8 |
| AA: Amidolytische Aktivität C_{Prot.}: Proteinkonzentration | | | | |

**Tabelle 4**

| 150 mM Na₂HPO₄/H₃PO₄, pH 6.0 10 mM TES 50 mg/ml Saccharose 0.01 % Tween 80® | | | | |
|---|---|---|---|---|
| | -20 °C | | -70 °C | |
| Tag | C_{Prot.} [mg/ml] | AA [MU/ml] | C_{Prot.} [mg/ml] | AA [MU/ml] |
| 0 | 5,9 | 3,5 | 5,9 | 3,5 |
| 1 | 5,5 | 3,5 | 5,6 | 3,4 |
| 2 | 5,6 | 3,4 | 5,5 | 3,5 |
| 5 | 6,0 | 3,5 | 6,2 | 3,6 |
| 9 | 6,0 | 3,3 | 6,0 | 3,3 |
| 15 | 6,0 | 3,6 | 5,9 | 3,1 |
| 29 | 5,9 | 3,5 | 6,0 | 3,6 |
| AA: Amidolytische Aktivität C_{Prot.}: Proteinkonzentration | | | | |

Ergebnis: Aus den Angaben in den Tabellen geht hervor, daß der Wirkstoff BM 06.022 in den genannten Formulierungen bei -20 °C und 4 °C mindestens 29 Tage stabil ist.

### Beispiel 5:

### Löslichkeit von r-PA in den erfindungsgemäßen Formulierungen

r-PA (BM 06.022) wurde über YM 10 auf 6 mg/ml konzentriert und gegen den unten ge- nannten Puffer dialysiert. Die Dialysate wurden wiederum über eine Amicon YM 10-Membran so lange konzentriert, bis eine Trübung auftrat. Nach Zentrifugation der Proben wurden die Überstände fünf Tage bei 4 °C gelagert. Zu Beginn und am Ende der Lagerung wurden die amidolytische Aktivität und C_{Prot.} bestimmt.

**Tabelle 5**

| 150 mM Na₂HPO₄/H₃PO₄, pH 6.0 10 mM TES 50 mg/ml Saccharose 0.01 % Tween 80® | | |
|---|---|---|
| Tag | C_{Prot.} [mg/ml] | AA [MU/ml] |
| 0 | 10,2 | 4,8 |
| 5 | 10,2 | 4,9 |
| AA: Amidolytische Aktivität C_{Prot.}: Proteinkonzentration | | |

Ergebnis: Die Löslichkeit von r-PA beträgt ca. 10 mg/ml. Bei der maximalen Proteinkonzentration kann die Probe bei 4 °C ohne Veränderung der amidolytischen Aktivität mindestens bis zu 5 Tagen gelagert werden.

### Beispiel 6

### Herstellung von flüssigen Darreichungsformen

Die folgenden Darreichungsformen wurden als Lösungen vor der Lyophilisation hergestellt:

Zusammensetzung der Lösungen vor der Lyophilisation

| Lösung A: | |
|---|---|
| BM 06.022 | 4 mg/ml |
| Na₂HPO₄ x 12 H₂O | 53,72 mg/ml |
| H₃PO₄ 85% | 11,3 mg/ml |
| Tranexamsäure | 1,6 mg/ml |
| Saccharose | 50 mg/ml |
| Tween 80®, pH 6 | 0,1 mg/ml |

| Lösung B: | |
|---|---|
| BM 06.022 | 4 mg/ml |
| K₂HPO₄ | 26,2 mg/ml |
| H₃PO₄ 85 % | 11,6 mg/ml |
| Tranexamsäure | 1,6 mg/ml |
| Saccharose | 70 mg/ml |
| Tween 80®, pH 6 | 0,1 mg/ml |

### Herstellung der Lyophilisate:

Nach Sterilfiltration werden jeweils 5 ml Aliquote in 20 ml Glasfläschen abgefüllt. Die Lyophilisation wird wie folgt durchgeführt: Die gefüllten Glasfläschen werden in einen Gefriertrockner gestellt und bei Temperaturen von - 40 bis - 50 °C Plattentemperatur für 10 Stunden bei Atmosphärendruck eingefroren. Anschließend wird an der Kammer ein Vakuumwert von p = 0,01 bis 0,1 mbar angelegt. Die Plattentemperatur wird danach so eingestellt, daß die Produkttemperatur zu jeder Zeit sicher unterhalb der jeweiligen Glasübergangstemperatur liegt. Nachdem die gesamte Eismenge wegsublimiert ist, wird die Plattentemperatur auf einen Wert von 20 - 40 °C erhöht und anschließend eine Nachtrocknung im Vakuum durchgeführt. Die Restfeuchte wurde nach üblichen Verfahren (Bestimmung nach der Methode von Karl Fischer) bestimmt und beträgt bei Lösung A 6 % und bei Lösung B 3 %. Die Einlagerung der Lyophilisate für Stabilitätsuntersuchungen erfolgt bei Temperaturen von 4 °C (Kühlschranktemperatur), 20 °C (Raumtemperatur) und 35 °C. Die Stabilität der Lyophilisate nach einer Einlagerungszeit von vier bis zwölf Wochen ist bei den angegebenen Temperaturen erfüllt, wie aus den analytischen Untersuchungen hinsichtlich der amidolytischen Aktivität und der Proteinkonzentration hervorgeht.

Rekonstitution der Lyophilisate für die Applikation: Die Lyophilisate werden mit Wasser für Injektionszwecke auf 10 ml aufgefüllt. Dies entspricht einer Verdünnung um den Faktor zwei im Verhältnis zum ursprünglichen Volumen der Lösung vor der Lyophilisation.

### Beispiel 7:

### Prüfung der Venenverträglichkeit der erfindungsgemäßen Formulierungen

Es wurden zwei Verum- und zwei Placebolösungen der in Tabelle 6 aufgeführten Zusammensetzungen zubereitet und bei Kaninchen intravenös verabreicht. Als Applikationsmenge wurden 0,5 ml/Tier verwendet, für jede der vier Testlösungen wurden fünf Tiere eingesetzt.

**Tabelle 6:**

| Zusammensetzung der Verum- und Placebolösungen | | |
|---|---|---|
| 1. Verumlösung | | |

| | Versuch Nr. 93/1292 | Versuch Nr. 93/1294 |
|---|---|---|
| BM06.022 | 10 MU | 10 MU |
| Di-Kaliumhydrogenphosphat | 131,0 mg | 131,0 mg |
| Phosphorsäure 85%ig | 58 mg | 58 mg |
| Tranexamsäure | 8,0 mg | 8,0 mg |
| Saccharose | 250,0 mg | 350,0 mg |
| Polysorbat 80 | 0,5 mg | 0,5 mg |
| Wasser für Injektionszwecke | ad 10,0 ml | ad 10,0 ml |
| pH | 6,0 | 6,0 |
| Osmolarität | 320 mOsmol | 351 mOsmol |

| | | |
|---|---|---|
| 2. Placebolösungen | | |

| | Versuch Nr. 93/1291 | Versuch Nr. 93/1293 |
|---|---|---|
| Di-Kaliumhydrogenphosphat | 131,0 mg | 131,0 mg |
| Phosphorsäure 85%ig | 58 mg | 58 mg |
| Tranexamsäure | 8,0 mg | 8,0 mg |
| Saccharose | 250,0 mg | 350,0 mg |
| Polysorbat 80 | 0,5 mg | 0,5 mg |
| Wasser für Injektionszwecke | ad 10,0 ml | ad 10,0 ml |
| pH | 6,0 | 6,0 |
| Osmolarität | 322 mOsmol | 343 mOsmol |

Ergebnis: Die Reaktion der Tiere bei der Injektion und die histologischen Befunde zeigen, daß die eingesetzten Prüflösungen sämtlich gut verträglich sind.

## Patentansprüche

1. Pharmazeutisches Präparat eines Plasminogenaktivators, das Zucker und Tranexamsäure als einzige Aminocarbonsäure enthält und kein Citrat beinhaltet.

2. Präparate gemäß Anspruch 1, **dadurch gekennzeichnet**, daß der Zucker ein Disaccharid, vorzugsweise Saccharose oder Trehalose, ist.

3. Präparat gemäß einem der Ansprüche 1 oder 2, **dadurch gekennzeichnet**, daß das Präparat als Lösung vorliegt und 40 - 100 mg/ml, vorzugsweise 50 - 70 mg/ml, Zucker enthält.

4. Präparat gemäß einem der Ansprüche 1 - 3, **dadurch gekennzeichnet**, daß das Präparat als Lösung vorliegt und 1 - 50 mMol/l, vorzugsweise 8 - 12 m Mol/l, Tranexamsäure enthält.

5. Präparat gemäß einem der Ansprüche 1 - 4, **dadurch gekennzeichnet**, daß das Präparat 0,005 - 0,1 % (w/v), vorzugsweise 0,01 % (w/v), Tensid enthält.

6. Präparat gemäß einem der Ansprüche 1 - 5, wobei der Plasminogenaktivator ein Protein vom Typ K2P, K1K2P, FK1K2P oder FK2K2P ist.

7. Präparat gemäß einem der Ansprüche 1 - 5, wobei der Plasminogenaktivator t-PA oder ein von t-PA durch Deletionen oder Substitutionen einzelner oder mehrerer Aminosäuren abgeleitetetes Derivat ist.

8. Präparat gemäß einem der Ansprüche 1, 2 oder 5 - 7, **dadurch gekennzeichnet**, daß die pharmazeutische Darreichungsform ein Lyophilisat ist.

9. Präparat gemäß einem der Ansprüche 1 - 7, **dadurch gekennzeichnet**, daß die Darreichungsform eine wässrige Lösung ist und der pH-Wert der Lösung 5,5 - 6,5 beträgt.

10. Verfahren zur Herstellung eines pharmazeutischen Präparats nach einem der Ansprüche 1 - 9, **dadurch gekennzeichnet**, daß man den Plasminogenaktivator zusammen mit Zucker und Tranexamsäure in eine geeignete pharmazeutische Darreichungsform überführt.

11. Verwendung einer Kombination der pharmazeutischen Hilfsstoffe Zucker, Phosphatpuffer, Tranexamsäure und Tensid zur Herstellung von venenverträglichen pharmazeutischen Darreichungsformen gemäß Anspruch 1.

12. Verwendung gemäß Anspruch 11 zur Herstellung von Darreichungsformen mit einer Lagerstabilität von mindestens zwei Jahren.

13. Verwendung einer Kombination der pharmazeutischen Hilfsstoffe Zucker und Tranexamsäure zur Langzeitstabilisierung von pharmazeutischen Darreichungsformen gemäß Anspruch 1, insbesondere zur Vermeidung des Verlustes der biologischen Aktivität des Plasminogenaktivators oder zur Vermeidung der Bildung von Aggregaten in pharmazeutischen Darreichungsformen enthaltend einen Plasminogenaktivator.

## Claims

1. Pharmaceutical preparation of a plasminogen activator which contains sugar and tranexamic acid as the only aminocarboxylic acid and which contains no citrate.

2. Preparations as claimed in claim 1, wherein the sugar is a disaccharide, preferably sucrose or trehalose.

3. Preparation as claimed in one of the claims 1 or 2, wherein the preparation is present as a solution and contains 40 - 100 mg/ml, preferably 50 - 70 mg/ml sugar.

4. Preparation as claimed in one of the claims 1 - 3, wherein the preparation is present as a solution and contains 1 - 50 mMol/l, preferably 8 - 12 mMol/l tranexamic acid.

5. Preparation as claimed in one of the claims 1 - 4, wherein the preparation contains 0.005 - 0.1 % (w/v), preferably 0.01 % (w/v) surfactant.

6. Preparation as claimed in one of the claims 1 - 5, wherein the plasminogen activator is a protein of the K2P, K1K2P, FK1K2P or FK2K2P type.

7. Preparation as claimed in one of the claims 1 - 5, wherein the plasminogen activator is t-PA or a derivative derived from t-PA by deletion or substitution of single or several amino acids.

8. Preparation as claimed in one of the claims 1, 2 or 5 - 7, wherein the pharmaceutical form of administration is a lyophilisate.

9. Preparation as claimed in one of the claims 1 - 7, wherein the form of administration is an aqueous solution and the pH value of the solution is 5.5 - 6.5.

10. Process for the production of a pharmaceutical preparation as claimed in one of the claims 1 - 9, wherein the plasminogen activator is converted together with sugar and tranexamic acid into a suitable pharmaceutical form of administration.

11. Use of a combination of the pharmaceutical auxiliary substances sugar, phosphate buffer, tranexamic acid and surfactant to produce pharmaceutical forms of administration which are tolerated by the veins as claimed in claim 1.

12. Use as claimed in claim 11 to produce forms of administration that have a storage stability of at least two years.

13. Use of a combination of the pharmaceutical auxiliary substances sugar and tranexamic acid for the long-term stabilization of pharmaceutical forms of administration as claimed in claim 1, in particular to prevent loss of the biological activity of the plasminogen activator or to prevent the formation of aggregates in pharmaceutical forms of administration containing a plasminogen activator.

## Revendications

1. Préparation pharmaceutique d'un activateur du plasminogène, qui contient un sucre et de l'acide tranexamique en tant que seul acide aminocarboxylique, et ne comporte pas de citrate.

2. Préparations selon la revendication 1, **caractérisées en ce que** le sucre est un disaccharide, de préférence le saccharose ou le tréhalose.

3. Préparation selon la revendication 1 ou 2, **caractérisée en ce que** la préparation se trouve sous forme d'une solution et contient 40-100 mg/ml, de préférence 50-70 mg/ml, de sucre.

4. Préparation selon l'une des revendications 1 à 3, **caractérisée en ce que** la préparation se trouve sous forme d'une solution et contient 1-50 mmoles/1, de préférence 8-12 mmoles/1, d'acide tranexamique.

5. Préparation selon l'une des revendications 1 à 4, **caractérisée en ce que** la préparation contient 0,005-0,1 % (p/v), de préférence 0,01 % (p/v), de tensioactif.

6. Préparation selon l'une des revendications 1 à 5, dans laquelle l'activateur du plasminogène est une protéine du type K2P, K1K2P, FK1K2P ou FK2K2P.

7. Préparation selon l'une des revendications 1 à 5, dans laquelle l'activateur du plasminogène est le t-PA (activateur tissulaire du plasminogène) ou un dérivé de t-PA obtenu par délétions ou remplacements d'un ou plusieurs aminoacides.

8. Préparation selon l'une des revendications 1, 2 ou 5-7, **caractérisée en ce que** la forme pharmaceutique de présentation est un lyophilisat.

9. Préparation selon l'une des revendications 1 à 7, **caractérisée en ce que** la forme de présentation est une solution aqueuse et le pH de la solution est 5,5-6,5.

10. Procédé pour la fabrication d'une préparation pharmaceutique selon l'une des revendications 1 à 9, **caractérisé en ce que** l'on convertit en une forme pharmaceutique de présentation appropriée l'activateur du plasminogène conjointement avec un sucre et de l'acide tranexamique.

11. Utilisation d'une association des adjuvants pharmaceutiques sucre, tampon phosphate, acide tranexamique et tensioactif, pour la préparation de formes pharmaceutiques de présentation selon la revendication 1, compatibles avec les veines.

12. Utilisation selon la revendication 11, pour la préparation de formes pharmaceutiques ayant une stabilité au stockage d'au moins deux ans.

13. Utilisation d'une association d'adjuvants pharmaceutiques sucre et acide tranexamique pour la stabilisation pendant de longues durées de formes pharmaceutiques de présentation selon la revendication 1, en particulier pour éviter la perte de l'activité biologique de l'activateur du plasminogène ou pour éviter la formation d'agrégats dans des formes pharmaceutiques de présentation contenant un activateur du plasminogène.
